(19) Europäisches Patentamt
European Patent Office
Office européen des brevets

(11) EP 4 806 628 A1

(12) **EUROPEAN PATENT APPLICATION**

(43) Date of publication:
**16.09.2026 Bulletin 2026/38**

(21) Application number: **25163391.3**

(22) Date of filing: **13.03.2025**

(51) International Patent Classification (IPC):
***B32B 5/02*** (2006.01) ***A61F 13/00*** (2024.01)
***A61F 13/49*** (2006.01) ***B32B 5/04*** (2006.01)
***B32B 5/08*** (2006.01) ***B32B 5/26*** (2006.01)
***B32B 7/022*** (2019.01) ***B32B 7/05*** (2019.01)
***D04H 3/005*** (2012.01) ***D04H 3/007*** (2012.01)
***D04H 13/00*** (2006.01)

(52) Cooperative Patent Classification (CPC):
**B32B 5/022; A61F 13/15; A61F 13/47; B32B 5/04; B32B 5/26; B32B 5/266; B32B 5/271; B32B 7/022; B32B 7/05; D04H 3/007; D04H 3/018; D04H 13/007;** B32B 5/08; B32B 5/267; B32B 5/268;
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fibertex Personal Care A/S**
**9220 Aalborg (DK)**

(72) Inventors:
- **TAIDAL, Lasse Kristoffer**
  **9220 Aalborg (DK)**
- **UDENGAARD, Brian**
  **9220 Aalborg (DK)**

(74) Representative: **Lorenz Seidler Gossel Part. mbB**
**Widenmayerstr. 23**
**80538 München (DE)**

(54) **HIGH TENSILE-STRENGTH NONWOVEN SHEETS**

(57) The invention relates to nonwoven sheets for use in the hygiene or medical industry. More specifically, the invention aims to reduce delamination and optimize tensile properties for nonwoven sheets that exceed a certain basis weight and calliper.

**Fig. 1**

10a
10a
10b
11
11
12
13
14

EP 4 806 628 A1

Processed by Luminess, 75001 PARIS (FR)

(Cont. next page)

Fig. 5

S
M
M
S
S

(52) Cooperative Patent Classification (CPC): (Cont.)
B32B 5/269; B32B 2250/03; B32B 2250/04;
B32B 2250/05; B32B 2250/20; B32B 2250/40;
B32B 2262/0215; B32B 2262/0253;
B32B 2262/124

## Description

The invention relates to nonwoven sheets for use in the hygiene or medical industry. More specifically, the invention aims to reduce delamination and optimize tensile properties for nonwoven sheets that exceed a certain basis weight and calliper.

The hygiene industry uses nonwoven sheets on a large scale as materials in hygiene products, such as baby diapers, adult incontinence products or femcare products, or medical products, like sterilization wraps, surgical gowns, or wound dressings. The nonwoven sheets have a textile feel, can be produced cheaply on a large scale and can be produced with a variety of different properties, depending on their intended use within the hygiene or medical products. For example, sheets for waist belts of diaper pants can advantageously have elastic properties, sheets for diaper back sheets will typically be required to have high liquid barrier properties, and so forth. In other cases, thicker sheets having relatively high tensile strength are desired.

Two different types of nonwoven materials can broadly be distinguished by the method of making and by its properties, namely staple fiber materials such as air laid or carded materials on the one hand, and endless fiber materials such as spunbonded and meltblown materials on the other hand. For various reasons, spunbonded materials are frequently used in today's hygiene and medical industry.

In many cases, the nonwoven sheets are formed from two or more layers of the same or different nonwoven materials to obtain desired properties. For example, elastic sheets can have an elastic material sandwiched between two spunbonded facing layers, as in EP 3 867 437 B1, or high barrier sheets can have a meltblown layer sandwiched between two spunbonded facing layers, as in EP 2 270 271 B1, or generally the sheets can comprise two or more spunbonded layers of different characteristic, for example, one loftier spunbonded layer for softness and one more compact spunbonded layer for improved tensile properties, as in EP 3 246 443 B1.

The nonwoven sheets, whether they are formed from one or more layers, are formed by laydown and subsequent bonding. While several bonding methods are available, calendering is often used in today's hygiene or medical industry. It involves the use of a two-roll nip consisting, in most cases, of a heated engraved roll (e-roll) and a typically also heated smooth roll (s-roll). The unbonded nonwoven material is fed to the calender nip and the fiber temperature is locally raised to the point at which tackiness and melting cause fiber segments caught between the tips of engraved points and the smooth roll to adhere together. The nonwoven materials bonded by calendering have a pattern of bonding points that holds the fibers of the material together and bonds the nonwoven layers into a cohesive sheet.

Whenever a multilayer nonwoven sheet reaches a basis weight or thickness above a certain threshold, however, in some cases a tendency of delamination of the individual layers has been observed when the sheets are exposed to some extend of stress. This limits the tensile strength of the nonwoven sheet, as the sheet consequently breaks in layers and not as a cohesive construction. A possible explanation for the problem is that, starting at a certain nonwoven thickness, it becomes challenging to compress the layers and heat the entire thickness of the nonwoven sheet in the calender. More specifically, there is typically a point where nip pressure and calender roll temperature cannot be anymore increased without risking the polymer sticking to the calender, necessitating line stops. Also, the nonwoven thickness may exceed the clearance of the pin height of the calender. The problem can be emphasized in sheets where the layers are formed from different polymers.

The prior art does not offer solutions to this problem that are satisfactory. Decreasing the line speed of the production line will give the nonwoven sheet more time in the nip of the calender, increase the time of the heat transfer from the calender to the nonwoven sheet and ultimately improve bonding, but it increases the cost of the production as the output in kg/hour decreases and the pin height of the embossed roll of the calender will be a continuously limiting factor.

There is hence a need for further solutions that reduce delamination and optimize tensile properties for nonwoven sheets that exceed a certain basis weight and calliper.

Against this background, the invention proposes a multilayer nonwoven sheet comprising at least three nonwoven material layers and having an overall basis weight of at least 30 gsm, wherein the sheet comprises a pattern of bonding points at which the fibers of the at least three layers are melt-bonded together to form a cohesive sheet, and wherein the fibers forming for a middle layer of the at least three layers comprise a thermoplastic elastomer.

The middle layer acts in the sense of an adhesive to the adjacent layers due to an inherent stickiness of freshly formed thermoplastic elastomer fibers. This improves bonding of the adjacent layers into a cohesive structure that does not delaminate when exposed to stress, resulting in an increased tensile strength for a given basis weight. Possible shortcomings cohesiveness at applicable bonding pressures, calender temperatures and line speeds can be compensated.

In embodiments, the overall basis weight of the sheet is at least 50 gsm, preferably at least 70 gsm, preferably at least 90 gsm. The upper limit for the overall basis weight of the sheet, in embodiments, can be 200 gsm, preferably 180 gsm, more preferably 160 gsm, and yet more preferably 140 gsm. In embodiments, the basis weight of one or both layers adjacent the middle layer greater 15 gsm, preferably greater 25 gsm, more preferably greater 40 gsm.

In embodiments, the overall calliper of the sheet is at least 0,30 mm. In embodiments, the calliper can be at least

0,50 mm, or at least 0,70 mm, or at least 0,90 mm. Reference is made to values as measured according to WSP120.6. These thicknesses may exceed the pin height of standard calender e-rolls.

In embodiments, the tensile MD @break of the sheet is at least 100 N/25 mm (WSP 110.4), or the CD tensile @break of the sheet is at least 60 N/25 mm (WSP 110.4), or both. In embodiments, the tensile MD @break per basis weight of the sheet is at least 1 N/25 mm/gsm (WSP 110.4), or the CD tensile @break per basis weight of the sheet is at least 0,6 N/25 mm/gsm (WSP 110.4), or both.

In embodiments, the middle layer is a meltblown layer. In embodiments, the basis weight of the middle layer accounts for 33% or less, preferably 25% or less, more preferably 20% or less, more preferably 15% or less, more preferably 10% or less, more preferably 7,5% or less, yet more preferably 5% or less of the overall basis weight of the sheet. In embodiments, the basis weight of the middle layer is smaller 10 gsm, preferably smaller 5 gsm. In the laminates of the invention, the function of the middle layer is increasing cohesiveness rather than imparting elastic properties.

As an alternative to a meltblown layer, the middle layer can also be another layer, for example a spunbonded layer, which comprises a thermoplastic elastomer.

In embodiments, the content of the thermoplastic elastomer in the fibers forming for the middle layer is at least 30 wt%, preferably at least 50 wt%, more preferably at least 70 wt.%, and yet more preferably at least 90 wt%. For sufficient stickiness, a certain minimum share of the material forming for the fibers of the middle layer is a thermoplastic elastomer. In an embodiment, the fibers of the middle layer essentially consist of the TPE, i.e. comprise more than 95 wt%, more than 99 wt% or 100 wt% thermoplastic elastomer. For processability, however, mixing with another thermoplastic polymer like a regular thermoplastic polypropylene can be of advantage. Especially in the case of meltblown fibers, the thermoplastic elastomer or the polymer mixture comprising the thermoplastic elastomer should have a relatively high melt flow rate. Preferred values are 75 g/10 min or more, 100 g/10 min or more, or 150 g/10 min or more (ISO 1133, 230°C, 2,16 kg).

In embodiments, the thermoplastic elastomer is a thermoplastic polyolefin elastomer (TPE-o), preferably a thermoplastic polyolefin elastomer comprising propylene-$\alpha$-olefin copolymers. Suitable TPE-o materials for use in the context of the present invention are comprised, for example, in the Vistamaxx™ series from ExxonMobil. An example comprises Vistamaxx™ 6902, which has a profile suitable for meltblowing applications. Alternatively or additionally, meaning as a mixture, other thermoplastic elastomer materials like especially thermoplastic polyurethanes (TPU) or styrenic block copolymers (TPE-s) may be used. In one embodiment, up to 20 wt.-% and preferably up to 10 wt.-% of a thermoplastic olefin, such as a homopolypropylene may be contained in the thermoplastic elastomer material next to the thermoplastic elastomer.

In embodiments, one or both layers adjacent to the middle layer are spunbonded layers. In embodiments, one or both of these layers can comprise crimped bicomponent fibers for increased softness and loft of the sheet. In embodiments, one or both of these layers can comprise monocomponent fibers for more compactness and higher tensile strength.

The fibers forming the layers adjacent to the middle layer may be formed from polyolefins. Preferred examples comprise polypropylene (PP), polyethylene (PE) and propylene-$\alpha$-olefin copolymers (co-PP), like propylene-ethylene copolymers The copolymer is preferably a poly(propylene-ethylene) copolymer. Melting temperatures of suitable polypropylene materials may range from 150-170°C, preferably 155-165°C. Melting temperatures of suitable propylene-$\alpha$-olefin copolymer materials may range from 140-160°C, preferably 145-155°C. Melting temperatures of suitable polyethylene materials may range from 130-150°C, preferably 135-145°C. All of the above melting temperatures are to be understood as referring to values determined with DSC according to ISO 11357-3. Typical values for melt flow rates of such polymers suitable for use in spunbonding may be 10-50 g/10min (ISO 1133-1, 230°C, 2.16 kg).

In embodiments, the middle layer as well as the layers adjacent to the middle layer may each be formed from more than one (sub-)layers. For example, the middle layer may comprise two meltblown (sub-)layers comprising a thermoplastic elastomer. As another example, one or both of the layers adjacent to the middle layer may comprise two spunbonded (sub-)layers. Hence, for meltblown middle layers, the invention comprises structures such as SMS, or SMMS, or SMMSS, or the like. For spunbonded middle layers, the invention comprises structures such as SSS, or SSSS, or the like.

In embodiments, at least one of the layers adjacent to the middle layer is formed from more than one (sub-)layers. In such cases, the effect of the invention may be especially emphasized because the line speed, at a given overall basis weight, will typically increase with an increasing number of layers. It is therefore contemplated that production lines with higher line speed (e.g. SSMMS lines) will benefit from this invention more, or already at lower basis weights, than lines with lower line speed (e.g. SMMS lines).

In embodiments, the layers adjacent to the middle layer differ from each other in regard of the fiber configuration, the polymers forming for the fibers of the layers, or both. For example, at least one of the layers adjacent to the middle layer can be formed from bicomponent fibers, preferably comprising a polypropylene component and a propylene-$\alpha$-olefin copolymer component. As another example, at least one of the layers adjacent to the middle layer can be formed from monocomponent fibers, preferably fibers formed from a polypropylene or fibers formed from a propylene-$\alpha$-olefin copolymer. Since the connection between adjacent layers can be worse for layers having different structure or chemistry, the effects of the invention can be particularly pronounced in such context.

The number of bonding points per square centimetre of fabric surface may be lower than 100 or lower than 70, and on the other hand may be higher than 30. The total area of the fabric surface taken up by the areal bonding points in one embodiment may be less than 18 % or less than 15 %, and on the other hand preferably higher than 8%.

The invention further relates to a method for making a nonwoven sheet according to the invention. The method can be an in-line process comprising the following in-line steps: providing a first fiber web forming for one of the layers adjacent to the middle layer; providing a second fiber web forming for the middle layer by depositing freshly formed endless fibers comprising the thermoplastic elastomer onto the first fiber web; providing a third fiber web forming for the other one of the layers adjacent to the middle layer by depositing freshly formed endless fibers onto the second fiber web or by associating a premade fiber web to the free surface of the second fiber web; and bonding the adjacent webs to form the nonwoven sheet by calendering.

Calendering involves the use of a two-roll nip consisting, in most cases, of a heated engraved roll (e-roll) and a typically also heated smooth roll (s-roll). The multi-layered fiber web is fed to the calender nip, typically by an apron leading to the calender nip, and the fiber temperature is locally raised to the point at which tackiness and melting cause fiber segments caught between the tips of engraved points and the smooth roll to adhere together.

The process temperature and, in particular, the temperature of the engraved calender roll is a significant parameter for bonding quality. For given polymers used in the webs, for a given nip pressure and line speed, the breaking strength reaches a maximum at a critical bonding temperature. These bonding temperatures, when using polymers like polypropylene and propylene-$\alpha$-olefin copolymers, can be between 140-180°C, for example (reference to the oil temperature of the oil that heats up the e-roll; the oil temperature for the s-roll temperature is typically some degrees Celsius lower). For high basis weight nonwoven materials, which are subject of the present invention, values in the upper half of these ranges, for example temperatures of 160-180°C. Nip pressures can lie between 50-150 N/mm, for example.

Typical line speeds can be between 50-600 m/min, preferably 75-350 m/min. In embodiments, the invention allows increasing the line speeds and outputs without detriment to delamination stability of the sheets, where the basis weight and thickness of the fabric would have otherwise advertised for lower line speeds. Hence, in an embodiment, line speeds above 200 m/min, 250 m/min or even 300 m/min can be preferred.

Yet further, the invention relates to a hygiene article or medical article comprising a nonwoven sheet according to the invention. The hygiene article can, for example, be a baby diaper, an adult incontinence product, or a femcare product. The medical article can, for example, be a sterilization wrap, surgical gown, or wound dressing.

Further details and advantages of the invention are discussed in the following with reference to embodiments and examples. The figures show:

Fig. 1: An exemplary process line for manufacturing SSMMS-type nonwoven sheets of the invention.

Fig. 2: A detailed illustration of a spunbonding machine.

Fig. 3: A detailed illustration of a meltblowing machine.

Fig. 4: Schematic illustrations of side-by-side (SBS), eccentric sheath-core (ECS) and trilobal bicomponent fiber configurations.

Fig. 5: A schematic cross section through an inventive SSMMS-type nonwoven sheet manufactured on a process line according to Fig. 1.

Fig. 6: An exemplary process line for manufacturing SMMS-type nonwoven sheets of the invention.

Fig. 7: A schematic cross section through an inventive SMMS-type nonwoven sheet manufactured on a process line according to Fig. 6.

Fig. 8: A stress-strain curve of the sheet of Comparative Example 12.

Fig. 9: A stress-strain curve of the sheet of Example 9.

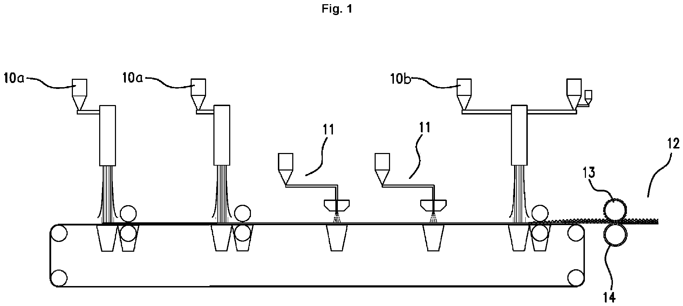

Fig. 1 shows an exemplary process line for manufacturing an SSMMS-type nonwoven fabric sheet. The process line comprises a conveyor belt that runs along two spunbonding machines 10a for forming a twin-layered SS bottom facing layer, two meltblowing machines 11 for forming a twin-layered MM middle layer, and another spunbonding machine 10b for forming a single-layered S top facing layer.

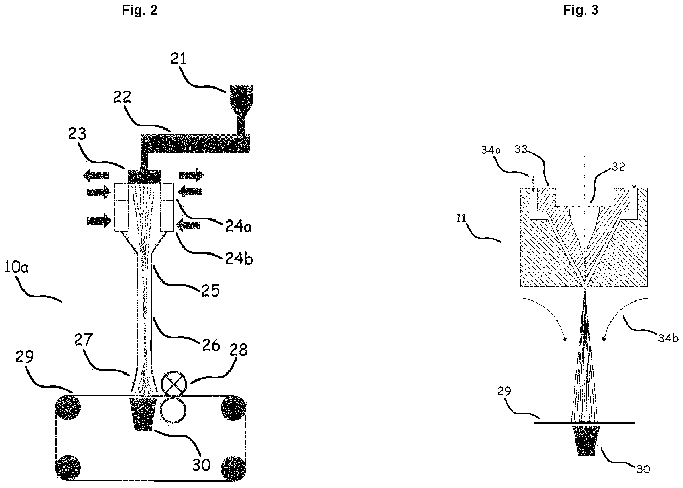

Fig. 2 illustrates the basic design of a spunbonding machine 10a. Thermoplastic polymers from a feed system 21 are provided to an extruder 22, heated and extruded through a spinneret 23 to form a curtain of filaments. A ventilation

system 24a draws monomer exhaust air from the spinneret 23 and a process air system 24b, including a pressurized cabin, directs process air to the curtain of filaments. The process air typically enters through a honeycomb system in order to have a good air distribution, to stabilize the curtain of filaments and to facilitate equal cooling and subsequent drawing of all filaments. Subsequent to the process air system 24b, the filaments and process air enter a stretching channel, comprising a so-called intermediate channel 25 and so-called SAS plates 26 for drawing and attenuating them. In intermediate channel 25, the air speed is increased, and the SAS plates 26 have an accurate position and precise distance, ensuring an equal airflow and drawing behaviour across the width of the machine. During the drawing and further cooling, the polymer chains within the filaments are oriented in the length direction of the filaments and slowly crystalize, resulting in an increased tenacity in each single resulting fiber. Subsequently, the thus finally stretched endless fibers enter a diffusor 27, also referred to as the distribution unit. Here, the airspeed and thereby the fiber speed is reduced and controlled to generate a turbulence that will randomize the orientation of the fibers. The combined fiber and air stream exiting the diffusor 27 is directed onto the movable and air-permeable spinbelt 29 for fiber laydown and the formation of a fibrous web. The web is consolidated by a pair of consolidation rolls 28. The process air is suctioned off by a suctioning unit 30 located below the spinbelt 29. The speed of the fibers exiting the diffusor 27 is higher than the travelling speed of the spinbelt 29, contributing to their random orientation not only in the airstream, but also in the web, with large proportions oriented in cross-machine direction. The ratio of speeds can be at least two-fold or at least three fold, with the speed ratio corresponding approximately to the average length of the endless fibers per one meter product length.

Fig. 3 illustrates the basic design of a meltblowing machine 11. Meltblowing is a comparatively simple single-step process that transforms polymer as a raw material into the web structure. Polymer is molten in an extruder and then passes a feed channel 32 and die holes in a spinneret 33 before going into a high-velocity hot air stream 24a that entangles and randomizes the filaments. Cooling air 34b mixes with the filament stream to cool and solidify the polymer before the fibers are laid onto the spinbelt 29. Like in spunbonding, the process air is suctioned off by a suctioning unit 30 located below the spinbelt 29.

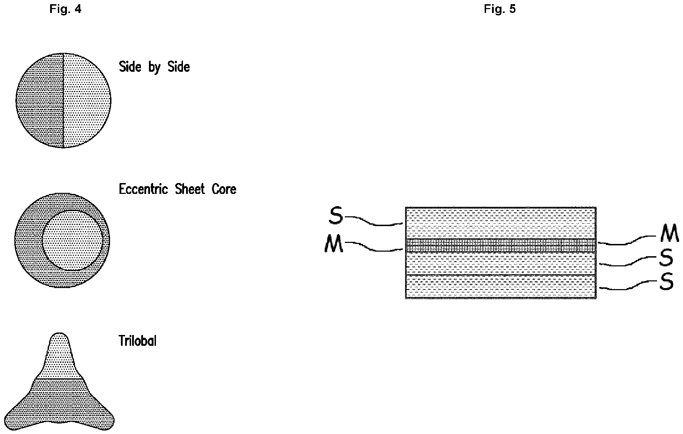

The spunbonding machine 11b for forming bicomponent fibers is of the same design as spunbonding machine 10a and hence a separate illustration is omitted. The difference is that a spunbonding machine for forming bicomponent fibers comprises two reservoirs for different polymer materials and independent polymer feeds for each material. The spinneret and die holes are designed for forming bicomponent filaments from the two components. The fibers are then drawn and attenuated into bicomponent fibers of a defined cross-sectional distribution of the two components. If the distribution is asymmetric and the components differ in their behavior during stretching and cooling, e.g. in their crystallization behavior, crimping of the fibers is observed. Fig. 4 shows schematic illustrations of typical asymmetric distribution of components in a bicomponent fiber, including side-by-side (SBS), eccentric sheath-core (ECS) and trilobal configurations.

Going back to Fig. 1, downstream of the spunbonding and meltblowing machines 10a, 11 and 10b, the web passes a calender bonding station 12 comprising a pair of calender rolls, e-roll 13 and s-roll 14, to bond the multilayered web into a multilayer nonwoven fabric sheet.

Fig. 5 shows a schematic cross-section of a sandwich-type stretchable nonwoven multilayer sheet comprising an SSMMS configuration, as made in a process line described above. The bottom facing layers is an SS type with two compact spunbonded layers of uncrimped monocomponent fibers. The middle layer is a MM type with two meltblown layers, which according to the invention are formed from a thermoplastic elastomer material. The top facing layer is an S type with one lofty spunbonded layer of crimped bicomponent fibers. The layers are hot calender point-bonded to each other.

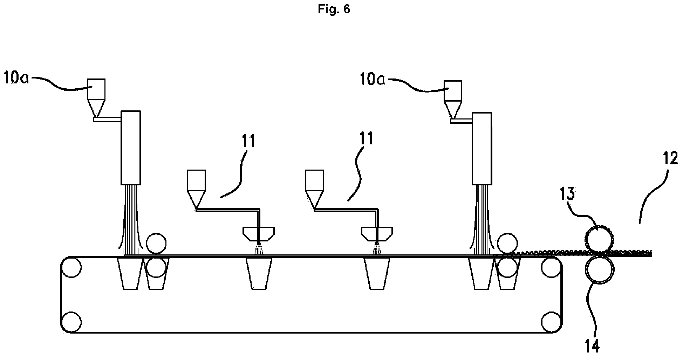

An exemplary process line for manufacturing an SMMS-type nonwoven fabric sheet is shown in Fig. 6. The setup is similar to the setup of Fig. 1, with the only difference that one of the spunbonding machines 10a in front of the meltblowing machines 11 is omitted and the spunbonding machine 10b (for making bicomponent fibers) after the meltblowing machine is replaced by a spunbonding machine 10a (for making monocomponent fibers).

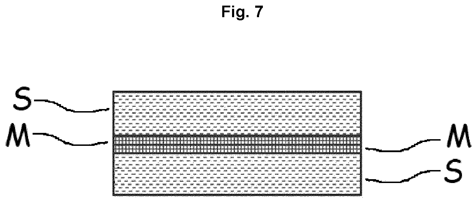

Fig. 7 shows a schematic cross-section of a sandwich-type stretchable nonwoven multilayer sheet comprising an SMMS configuration, as made in a process line as per Fig. 6. The bottom facing layer is an S type with one compact spunbonded layer of uncrimped monocomponent fibers. The middle layer is a MM type with two meltblown layers, which according to the invention are formed from a thermoplastic elastomer material. The top facing layer is another S type with one compact spunbonded layer of uncrimped monocomponent fibers. The layers are hot calender point-bonded to each other.

<u>Examples 1-10 and Comparative Examples 11-12:</u>

Examples 1-10 and Comparative Examples 11-12 as described in Table 1 were prepared on a SMMS line as described in connection with Fig. 6 and have a structure as shown in Fig. 7. The S bottom facing layer and the S top facing layer are both of monocomponent PP fibers (Borealis HG475FB). The bond pattern was an open dot bond pattern with a bonding area of 13,6%.

Table 1

| | Basis weight (gsm) | | | | | Spunbond / meltblown ratio | Vistamaxx 6902 / HL708FB ratio in M layers |
|---|---|---|---|---|---|---|---|
| | Overall | 1st S layer | 1st M layer | 2nd M layer | 2nd S layer | | |
| Ex 1 | 100 | 45 | 5 | 5 | 45 | 90/10 | 40/60 |
| Ex 2 | 100 | 47,5 | 2,5 | 2,5 | 47,5 | 95/5 | 40/60 |
| Ex 3 | 100 | 45 | 5 | 5 | 45 | 90/10 | 60/40 |
| Ex 4 | 100 | 47,5 | 2,5 | 2,5 | 47,5 | 95/5 | 60/40 |
| Ex 5 | 100 | 45 | 5 | 5 | 45 | 90/10 | 80/20 |
| Ex 6 | 100 | 47,5 | 2,5 | 2,5 | 47,5 | 95/5 | 80/20 |
| Ex 7 | 100 | 45 | 5 | 5 | 45 | 90/10 | 90/10 |
| Ex 8 | 100 | 47,5 | 2,5 | 2,5 | 47,5 | 95/5 | 90/10 |
| Ex 9 | 100 | 45 | 5 | 5 | 45 | 90/10 | 100/0 |
| Ex 10 | 100 | 47,5 | 2,5 | 2,5 | 47,5 | 95/5 | 100/0 |
| C Ex 11 | 100 | 45 | 5 | 5 | 45 | 90/10 | 0/100 |
| C Ex 12 | 100 | 47,5 | 2,5 | 2,5 | 47,5 | 95/5 | 0/100 |

Table 1 (cntd.)

| | Line speed [m/min] | Embossed roller [°C] | Smooth roller [°C] | Nip pressure [N/mm] |
|---|---|---|---|---|
| Ex 1 | 78 | 169 | 159 | 71 |
| Ex 2 | 74 | 169 | 159 | 71 |
| Ex 3 | 78 | 169 | 159 | 71 |
| Ex 4 | 74 | 169 | 159 | 71 |
| Ex 5 | 78 | 169 | 159 | 71 |
| Ex 6 | 74 | 169 | 159 | 71 |
| Ex 7 | 78 | 169 | 159 | 71 |
| Ex 8 | 74 | 169 | 159 | 71 |
| Ex 9 | 78 | 169 | 159 | 71 |
| Ex 10 | 74 | 169 | 159 | 71 |
| C Ex 11 | 78 | 169 | 159 | 71 |
| C Ex 12 | 74 | 169 | 159 | 71 |

The Borealis HL708FB polymer is a polypropylene homopolymer. The melting temperature is 158°C (ISO 11357-3) and the melt flow rate at 230°C is 800 g/10 min (ISO 1133-1; 2,16 kg), making it suitable for meltblown applications.

The Borealis HG475FB polymer is a polypropylene homopolymer with narrow. The melting temperature is 161°C (ISO 11357-3) and the melt flow rate at 230°C is 27 g/10 min (ISO 1133-1; 2,16 kg), making it suitable for spunbonding.

The Exxon Mobile Vistamaxx™ 6902 polymer is a propylene elastomer primarily composed of isotactic propylene repeat units with 12% ethylene content and random ethylene distribution. The melt flow rate is 43 g/10 min at 190°C and 100 g/10 min at 230°C (ISO 1133-1; 2,16 kg), making is suitable for meltblowing applications.

Polymers and test methods are as specified above. The resulting sheets are again tested for machine-directional (MD) and cross machine-directional (CD) tensile strength @break (WSP 110.4). The results on are shown in Table 2.

Table 2

| | Tensile @break MD [N/25mm] (n=15) | Tensile @break CD [N/25mm] (n=15) |
|---|---|---|
| Ex 1 | 102,1 | 52,2 |
| Ex 2 | 97,1 | 51,6 |
| Ex 3 | 104,4 | 56,5 |
| Ex 4 | 94,8 | 53 |
| Ex 5 | 106,2 | 60,9 |
| Ex 6 | 98,9 | 57,7 |
| Ex 7 | 105,2 | 60,2 |
| Ex 8 | 92,6 | 56,8 |
| Ex 9 | 104,9 | 61,6 |
| Ex 10 | 100,9 | 60,9 |
| C Ex 11 | 95,4 | 44,7 |
| C Ex 12 | 93,6 | 46,6 |

The data again show increasing tensile strength for both MD and CD with increasing addition of Vistamaxx 6902. Specifically, when comparing Example 9 and Comparative Example 12, the exchange of the MM material from PP to TPE-o led to a significant increase of tensile @break in MD and even more significant in CD.

The MM middle layer does not in itself significantly contribute to the tensile strength. If any, one would expect tensile strength to decrease if an elastic material is added to a layer instead of an inelastic material. The increase in tensile strength in the examples can hence be attributed to an improved resistance against delamination under stress.

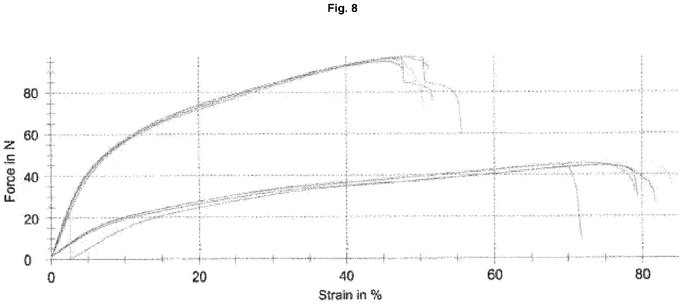

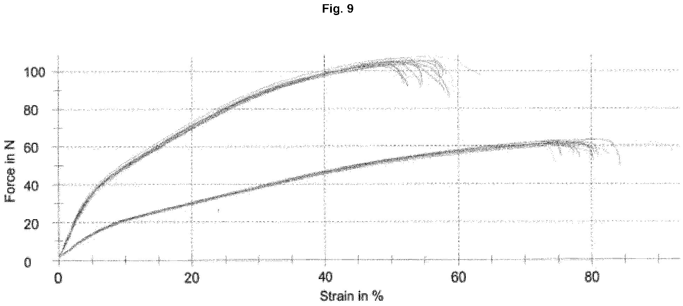

The resistance against delamination is also illustrated in the stress-strain curves of Fig. 8-9. Fig. 8 shows a stress-strain curve of Comparative Example 12. At about 45% elongation, the material breaks in MD at about 95 N/mm. A step-shape of the curve in the region showing the MD break demonstrates a delamination of the materials at break. Fig. 9 shows an identical illustration for Example 9. Here, the step-shape is absent when the material breaks in MD at about 45% elongation and about 105 N/mm.

## Claims

1. A multilayer nonwoven sheet comprising at least three nonwoven material layers and having an overall basis weight of at least 30 gsm, wherein the sheet comprises a pattern of bonding points at which the fibers of the at least three layers are melt-bonded together to form a cohesive sheet, and wherein the fibers forming for a middle layer of the at least three layers comprise a thermoplastic elastomer.

2. The multilayer nonwoven sheet according to claim 1, wherein the overall basis weight of the sheet is at least 50 gsm, preferably at least 70 gsm, more preferably at least 90 gsm.

3. The multilayer nonwoven sheet according to any one of the preceding claims, wherein the overall calliper of the sheet is at least 0,30 mm, preferably at least 0,50 mm, more preferably at least 0,70 mm (WSP120.6).

4. The multilayer nonwoven sheet according to any one of the preceding claims, wherein the tensile MD @break per basis weight of the sheet is at least 1 N/25 mm/gsm (WSP 110.4), or the CD tensile @break per basis weight of the sheet is at least 0,6 N/25 mm/gsm (WSP 110.4), or both.

5. The multilayer nonwoven sheet according to any one of the preceding claims, wherein the middle layer is a meltblown layer.

6. The multilayer nonwoven sheet according to any one of the preceding claims, wherein the basis weight of the middle layer accounts for 33% or less, preferably 20% or less, more preferably 10% or less, yet more preferably 5% or less of the overall basis weight of the sheet.

7. The multilayer nonwoven sheet according to any one of the preceding claims, wherein the content of the thermoplastic elastomer in the fibers forming for the middle layer is at least 30 wt%, preferably at least 50 wt%, more preferably at least 70 wt.%, and yet more preferably at least 90 wt%.

8. The multilayer nonwoven sheet according to any one of the preceding claims, wherein the thermoplastic elastomer is a thermoplastic polyolefin elastomer (TPE-o), preferably a thermoplastic polyolefin elastomer comprising propylene-$\alpha$-olefin copolymers.

9. The multilayer nonwoven sheet according to any one of the preceding claims, wherein one or both layers adjacent to the middle layer are spunbonded layers.

10. The multilayer nonwoven sheet according to any one of the preceding claims, wherein the layers adjacent to the middle layer differ from each other in regard of the fiber configuration, the polymers forming for the fibers of the layers, or both.

11. The multilayer nonwoven sheet according to any one of the preceding claims, wherein at least one of the layers adjacent to the middle layer is formed from bicomponent fibers, preferably comprising a polypropylene component and a propylene-$\alpha$-olefin copolymer component.

12. The multilayer nonwoven sheet according to any one of the preceding claims, wherein at least one of the layers adjacent to the middle layer is formed from monocomponent fibers, preferably fibers formed from a polypropylene or fibers formed from a propylene-$\alpha$-olefin copolymer.

13. A method for making a nonwoven sheet according to any one of the preceding claims, comprising the following in-line steps:

providing a first fiber web forming for one of the layers adjacent to the middle layer;
providing a second fiber web forming for the middle layer by depositing freshly formed endless fibers comprising the thermoplastic elastomer onto the first fiber web;
providing a third fiber web forming for the other one of the layers adjacent to the middle layer by depositing freshly formed endless fibers onto the second fiber web or by associating a premade fiber web to the free surface of the second fiber web; and
bonding the adjacent webs to form the nonwoven sheet by calendering.

14. A hygiene article comprising a nonwoven sheet according to any one of claims 1-12.

15. A medical article comprising a nonwoven sheet according to any one of claims 1-12.

Fig. 1

10a
10a
10b
11
11
12
13
14

Fig. 2

21
22
23
24a
24b
25
10a
26
27
28
29
30

Fig. 3

34a
33
32
11
34b
29
30

Fig. 4
Side by Side
Eccentric Sheet Core
Trilobal
Fig. 5
S
M
M
S
S

Fig. 6

10a
10a
11
11
12
13
14

Fig. 7

S
M
M
S

Fig. 8

80
60
40
20
0
Force in N
0
20
40
60
80
Strain in %

Fig. 9

Force in N
100
80
60
40
20
0
0
20
40
60
80
Strain in %

Europäisches Patentamt
European Patent Office
Office européen des brevets

# EUROPEAN SEARCH REPORT

Application Number
EP 25 16 3391

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/207280 A1 (ERLANDSSON SVEN KRISTER [US] ET AL) 21 July 2016 (2016-07-21) | 1,2,5,7, 8,11,13, 14 | INV. B32B5/02 A61F13/00 A61F13/49 B32B5/04 B32B5/08 B32B5/26 B32B7/022 B32B7/05 D04H3/005 D04H3/007 D04H13/00 |
| Y | * paragraphs 3, 81, 82, 94; Table 3 * | 4,10 | |
| X | US 2005/148260 A1 (KOPACZ THOMAS J [US] ET AL) 7 July 2005 (2005-07-07) | 1-3,5-8, 14 | |
| Y | * paragraphs 1, 4, 8, 33, 35, 47, 50, 55, 87-89, 91, 92, 95, 99, 106; examples 1, 4 * | 4,10 | |
| X | US 6 103 647 A (SHULTZ JAY SHELDON [US] ET AL) 15 August 2000 (2000-08-15) | 1,2,5, 7-9, 11-15 | |
| Y | * column 12, line 48 - column 13, line 67; examples 1, 2; claims 1, 3, 4, 9, 12-15, 23, 24 * | 4,10 | |
| X | US 2009/068420 A1 (PASCAVAGE PETER W [US]) 12 March 2009 (2009-03-12) | 1,2,5-9, 11,13,14 | |
| Y | * paragraphs 37, 49-58; claims * | 4,10 | **TECHNICAL FIELDS SEARCHED (IPC)** |
| X | US 2010/124864 A1 (DHARMARAJAN RAJA N [US] ET AL) 20 May 2010 (2010-05-20) * paragraph 53 * | 1,10 | B32B A61F D04H |
| Y | US 2018/368647 A1 (CHANG YU-WEN [US] ET AL) 27 December 2018 (2018-12-27) * paragraph 82 * | 10 | |
| Y | US 9 072 633 B2 (AUTRAN JEAN-PHILIPPE MARIE [US]; ROE DONALD CARROLL [US] ET AL.) 7 July 2015 (2015-07-07) * Table 6B * | 4 | |
| Y | WO 2023/083600 A1 (FIBERTEX PERSONAL CARE AS [DK]) 19 May 2023 (2023-05-19) * Table 3 * | 4 | |

The present search report has been drawn up for all claims

2

| Place of search | Date of completion of the search | Examiner |
|---|---|---|
| The Hague | 25 August 2025 | Barenbrug, Theo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 16 3391

This annex lists the patent family members relating to the patent documents cited in the above-mentioned European search report. The members are as contained in the European Patent Office EDP file on
The European Patent Office is in no way liable for these particulars which are merely given for the purpose of information.

25-08-2025

| Patent document cited in search report | Publication date | Patent family member(s) | Publication date |
|---|---|---|---|
| US 2016207280 A1 | 21-07-2016 | US 2016207280 A1 | 21-07-2016 |
| | | WO 2016118497 A1 | 28-07-2016 |
| US 2005148260 A1 | 07-07-2005 | AU 2004311996 A1 | 21-07-2005 |
| | | BR PI0405434 A | 30-08-2005 |
| | | EP 1703833 A2 | 27-09-2006 |
| | | KR 20060129233 A | 15-12-2006 |
| | | US 2005148260 A1 | 07-07-2005 |
| | | WO 2005065517 A2 | 21-07-2005 |
| US 6103647 A | 15-08-2000 | AR 006097 A1 | 11-08-1999 |
| | | AU 705599 B2 | 27-05-1999 |
| | | BR 9707985 A | 27-07-1999 |
| | | CA 2247169 A1 | 18-09-1997 |
| | | CN 1217759 A | 26-05-1999 |
| | | CO 4820421 A1 | 28-07-1999 |
| | | DE 69723820 T2 | 19-05-2004 |
| | | EP 0886690 A1 | 30-12-1998 |
| | | ID 19782 A | 30-07-1998 |
| | | KR 19990087750 A | 27-12-1999 |
| | | TW 352367 B | 11-02-1999 |
| | | US 6103647 A | 15-08-2000 |
| | | WO 9734037 A1 | 18-09-1997 |
| | | ZA 97726 B | 01-08-1997 |
| US 2009068420 A1 | 12-03-2009 | CN 101848807 A | 29-09-2010 |
| | | EP 2190658 A1 | 02-06-2010 |
| | | ES 2395884 T3 | 15-02-2013 |
| | | HK 1148984 A1 | 23-09-2011 |
| | | JP 5563459 B2 | 30-07-2014 |
| | | JP 2010538181 A | 09-12-2010 |
| | | TW 200918702 A | 01-05-2009 |
| | | US 2009068420 A1 | 12-03-2009 |
| | | WO 2009032868 A1 | 12-03-2009 |
| US 2010124864 A1 | 20-05-2010 | CN 105291495 A | 03-02-2016 |
| | | US 2010124864 A1 | 20-05-2010 |
| US 2018368647 A1 | 27-12-2018 | AU 2016361953 A1 | 07-06-2018 |
| | | AU 2016362322 A1 | 07-06-2018 |
| | | BR 112018009642 A2 | 06-11-2018 |
| | | BR 112018009724 A2 | 06-11-2018 |
| | | CA 3005607 A1 | 08-06-2017 |
| | | CN 108350238 A | 31-07-2018 |
| | | CN 109070554 A | 21-12-2018 |
| | | EP 3383649 A1 | 10-10-2018 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 25 16 3391

This annex lists the patent family members relating to the patent documents cited in the above-mentioned European search report. The members are as contained in the European Patent Office EDP file on
The European Patent Office is in no way liable for these particulars which are merely given for the purpose of information.

25-08-2025

| Patent document cited in search report | Publication date | Patent family member(s) | Publication date |
|---|---|---|---|
| | | GB 2556859 A | 06-06-2018 |
| | | KR 20180080283 A | 11-07-2018 |
| | | KR 20180080326 A | 11-07-2018 |
| | | US 2018353047 A1 | 13-12-2018 |
| | | US 2018368647 A1 | 27-12-2018 |
| | | WO 2017095483 A1 | 08-06-2017 |
| | | WO 2017096047 A1 | 08-06-2017 |
| US 9072633 B2 | 07-07-2015 | CA 2654619 A1 | 13-12-2007 |
| | | EP 2023876 A2 | 18-02-2009 |
| | | JP 4987972 B2 | 01-08-2012 |
| | | JP 2009539460 A | 19-11-2009 |
| | | US 2007287348 A1 | 13-12-2007 |
| | | WO 2007141745 A2 | 13-12-2007 |
| WO 2023083600 A1 | 19-05-2023 | CN 118055748 A | 17-05-2024 |
| | | EP 4373450 A1 | 29-05-2024 |
| | | JP 7600467 B2 | 16-12-2024 |
| | | JP 2024535322 A | 30-09-2024 |
| | | KR 20240045381 A | 05-04-2024 |
| | | MY 204431 A | 28-08-2024 |
| | | US 2025122654 A1 | 17-04-2025 |
| | | WO 2023083600 A1 | 19-05-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3867437 B1
- EP 2270271 B1
- EP 3246443 B1